# EUROPÄISCHE PATENTSCHRIFT

(11) **EP 2 787 934 B1**
(45) Veröffentlichungstag und Bekanntmachung des Hinweises auf die Patenterteilung: **19.07.2017**
(21) Anmeldenummer: 12809597.3
(22) Anmeldetag: 10.12.2012
(51) Int. Cl.: A61F 2/91

(54) **KÖRPERIMPLANTAT MIT MARKERELEMENT**
BODY IMPLANT HAVING A MARKER ELEMENT
IMPLANT CORPOREL COMPRENANT UN ÉLÉMENT MARQUEUR

(30) Priorität: 16.03.2012 DE 102012005356
(43) Veröffentlichungstag der Anmeldung: 15.10.2014
(73) Patentinhaber: Admedes Schuessler GmbH, 75179 Pforzheim (DE)
(72) Erfinder: SCHRADER, Bernd, 75169 Remchingen (DE); HEGEL, Alexander, 76187 Karlsruhe (DE)
(74) Vertreter: Müller-Boré & Partner Patentanwälte PartG mbB
(86) Internationale Anmeldenummer: PCT/EP2012/005093
(87) Internationale Veröffentlichungsnummer: WO 2013/135260

(56) Entgegenhaltungen:
- US-A1- 2006 222 756
- US-A1- 2007 156 230
- US-A1- 2007 266 542
- US-B1- 6 402 777

## Beschreibung

Die vorliegende Erfindung betrifft ein Körperimplantat, insbesondere einen Stent zum Einführen in einen lebenden Körper, mit einer guten Röntgensichtbarkeit sowie auf ein Verfahren zum Herstellen eines derartigen Körperimplantats.

Körperimplantate bzw. Stents dieser Art schützen Kanäle lebender Körper wie beispielsweise Blutgefäße, Speiseröhre, Harnröhre oder Nierengänge durch Einführen des Stents und Expandieren des Stents im Inneren des Körperkanals. Auf diese Weise kann ein Kollabieren oder Verschließen des jeweiligen Körperkanals verhindert werden. Darüber hinaus wird ein Stent beispielsweise für intercerebrale Gefäßaussackungen, sogenannte Aneurysmen eingesetzt, die die häufigste Ursache für nicht-traumatische Subarachnoidal-Blutungen sind. Ihre Inzidenz liegt in der Gesamtbevölkerung bei 1%, nach Autopsiestudien sogar bis zu 9%. Patomorphologisch sind intercerebrale Aneurysmen in der Regel echte, sacurale Aneurysmen, die meist in Gefäßaufzweigungen lokalisiert sind (vgl. beispielsweise Schumacher M. "Diagnostic work-up in cerebral aneurysms" in Nakstadt PHj (ed): "cerebral aneurysms", pp 13-24, Bologna: Centauro (2000)).

Darüber hinaus können derartige Körperimplantate bzw. Stents als Träger von Medikamenten dienen, um eine lokale Therapie innerhalb des Körperkanals zu ermöglichen.

Diese Stents werden in einem kollabierten Zustand in einen Körperkanal eingeführt und nach Anordnung des Stents in dem Körperkanal expandiert. Diese Stents bestehen üblicherweise aus rostfreiem Edelstahl oder einer Kobalt-Chrom-Tantal-Legierung. Die Stents werden bevorzugt durch eine Aufweitungseinrichtung, wie beispielsweise einem Ballonkatheter, in den Körperkanal eingeführt und dort aufgeweitet.

Die Stents können jedoch auch aus anderen Werkstoffen wie beispielsweise Polymeren, selbstabbaubaren Werkstoffen wie beispielsweise Milchsäure-Werkstoffen bzw. Derivaten sowie aus Nitinol (Nickel-Titan-Legierungen) und/oder aus anderen selbstexpandierbaren Werkstoffen wie beispielsweise sogenannten Formgedächtnis-Werkstoffen bestehen.

Um die Röntgensichtbarkeit von diesen Stents zu erhöhen, werden diese vielfach mit zusätzlichen Elementen (sogenannten Markern) versehen, die aus einem Material mit hoher Röntgensichtbarkeit hergestellt sind.

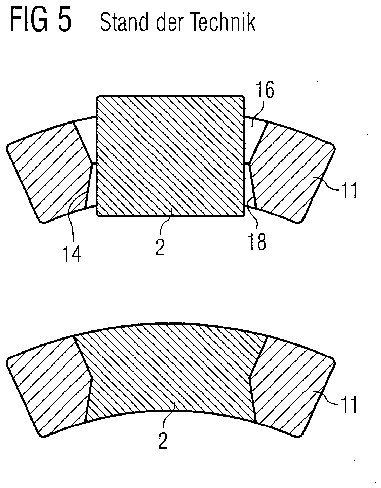

DE 10 2005 019 612 B4 beschreibt die mechanische Verriegelung eines Röntgenmarkers mittels eines Doppelkegels des Ausschnitts. Dabei wird zunächst, wie in Fig. 5 gezeigt ist, ein Innenkonus 14 an der Implantatstruktur 11 angebracht. Danach wird das zylindrische Markerelement 2 in den Ausschnitt so eingesetzt, daß das Markerelement 2 sowohl auf einer Innenseite als auch einer Außenseite des Körperimplantats vorsteht. Aufgrund der Zylinderform des Markenelements 2 und der Doppelkonus- bzw. Kegelform des Ausschnitts ergibt sich jeweils auf der Außenseite als auch auf der Innenseite des Körperimplantats ein Ringspalt 16, 18 zwischen dem Markerelement 2 und der Implantatstruktur 11. Dieser Ringspalt 16, 18 dient der Aufnahme des Materials des Markerelements 2 beim Verpressen bzw. Vernieten des Markerelements 2.

US 2007/0266542 A1 offenbart einen röntgensichtbaren Marker für eine intraluminale medizinische Vorrichtung, bei dem eine röntgensichtbare Niete in einem Ausschnitt eines Eyelets verpresst ist.

US 2006/0222756 A1 offenbart medizinische Vorrichtungen, Medikamentenbeschichtungen und Verfahren zum Erhalten der Medikamentenbeschichtungen.

US 6 402 777 B1 beschreibt röntgensichtbare Stentmarker in der Gestalt von verpressten Nieten.

Die Aufgabe der Erfindung besteht in der Schaffung eines Körperimplantats mit einer guten Röntgensichtbarkeit, das einfach und kostengünstig hergestellt werden kann, sowie eines Verfahrens zum Herstellen eines derartigen Körperimplantats.

Diese Aufgabe wird durch ein Körperimplantat mit den Merkmalen nach Anspruch 1, ein Verfahren mit den Merkmalen nach Anspruch 7 gelöst. Vorteilhafte Weiterbildungen der Erfindung sind in den abhängigen Ansprüchen definiert.

Gemäß einem Gesichtspunkt wird ein Körperimplantat geschaffen, insbesondere ein Stent, zum Einführen oder Implantieren in einen lebenden Körper mit einem Markerelement aus einem röntgensichtbaren Material , das in einen Ausschnitt oder eine Öffnung oder eine Aushöhlung einer Implantatstruktur eingesetzt ist,
wobei zumindest ein Rand des Ausschnitts bereichsweise derart verformt bzw. verpresst ist, dass das Markerelement formschlüssig von dem Ausschnitt aufgenommen ist, indem sich verformte Ränder und nicht verformte Ränder entlang des Umfangs des Ausschnitts abwechseln.

Dabei bildet der Ausschnitt bzw. die Aushöhlung vorzugsweise auf einer Seite einen Formschluss durch die Form des Ausschnitts bzw. der Aushöhlung. Dies wird erreicht, indem der Ausschnitt beispielsweise eine konische Form bzw. eine Kegelform aufweist. Eine weitere Möglichkeit besteht darin, die Aushöhlung auf einer Seite mit einem Boden zu versehen, diese also als Sackloch auszubilden.

Durch das bereichsweise Verpressen des Rands der Implantatstruktur auf der offenen Seite bzw. der Seite des Einsetzens des Markerelements, wird ein Fixieren des Markerelements auf einfache Weise erzielt.

Vorzugsweise ist der Ausschnitt im wesentlichen konisch und der Rand mit dem größeren Durchmesser ist verpresst, um das Markerelement zu fixieren.

Weiter bevorzugt ist der Rand des Ausschnitts bzw. der Aushöhlung an mehreren Stellen im wesentlichen punktförmig verformt. Dies bedeutet, dass eine Art Körnung im Bereich des Rands vorgenommen wird, um ein Fließen von Material in den Bereich des Ausschnitts hinein zu erzeugen. Auf diese Weise wird das Markerelement auf einfache Weise in dem Ausschnitt bzw. der Aushöhlung fixiert. Hierzu sollten mindestens zwei Körnungen um 180° versetzt am Rand erzeugt werden, vorzugsweise drei Körnungen bzw. Verpressungen um 120° versetzt.

Vorzugsweise ist das Markerelement vor dem Einpressen im wesentlichen zylindrisch und weist nach dem Einpressen im Querschnitt eine Kreisbogenform in Übereinstimmung mit dem Ausschnitt auf. Alternativ weist das Markerelement vor dem Einpressen bereits die Kreisbogenform auf, indem es beispielsweise aus einem Rohr ausgeschnitten wird.

Weiter bevorzugt ist das Markerelement nach dem Einpressen bzw. Einnieten innen/außen im wesentlichen eben mit der Implantatstruktur, um Verletzungen eines Körperkanals zu vermeiden, in den das Körperimplantat eingeführt wird.

Vorzugsweise weist das Markerelement ein Material mit hoher Absorption für Röntgenstrahlen, wie beispielsweise Gold, Platin, Tantal, Platinlegierung, Platin-Iridium, Niob, auf.

Gemäß einem weiteren Gesichtspunkt wird ein Verfahren zum Herstellen eines Körperimplantats, insbesondere eines Stents, zur Verfügung gestellt, das folgende Schritte aufweist:
Formen eines Ausschnitts bzw. Lochs bzw. Aushöhlung, Sackloch etc. in einer Implantatstruktur;
Einsetzen eines Markerelements in den Ausschnitt bzw. die Aushöhlung; und
Verformen bzw. Verpressen eines Rands des Ausschnitts bzw. der Aushöhlung bereichsweise derart, dass das Markerelement formschlüssig von dem Ausschnitt bzw. der Aushöhlung aufgenommen wird, indem sich verformte Ränder und nicht verformte Ränder entlang des Umfangs des Ausschnitts abwechseln.

Vorzugsweise weist das Verfahren folgende Schritt auf:
Formen des Ausschnitts im wesentlichen in einer konischen Gestalt; und
Verpressen des Rands mit dem größeren Durchmesser, um das Markerelement zu fixieren.

Weiter bevorzugt weist das Verfahren des weiteren den Schritt des punktförmigen Verformens des Rands an mehreren Stellen, vorzugsweise an drei Stellen in im wesentlichen gleichen Winkelabständen auf.

Vorzugsweise wird das Markerelement aus einem Rohr ausgeschnitten, so dass es bereits vor dem Einsetzen und Vernieten die Form des kreisbogenförmigen Ausschnitts aufweist.

Nachfolgend werden Ausführungsbeispiele der Erfindung anhand schematischer Zeichnungen näher erläutert.
Fig. 1 zeigt einen Abschnitt eines Körperimplantats mit einen in einen Ausschnitt eingesetzten Markerelement.
Fig. 2 zeigt eine Draufsicht sowie Schnittansichten des in Fig. 1 gezeigten Abschnitts eines Körperimplantats.
Fig. 3 zeigt wie das Markerelement in den Ausschnitt eingesetzt und dort fixiert wird.
Fig. 4 zeigt eine Abwandlung des Markerelements von Fig. 3.
Fig. 5 zeigt wie ein Markerelement gemäß dem Stand der Technik verpresst wird.

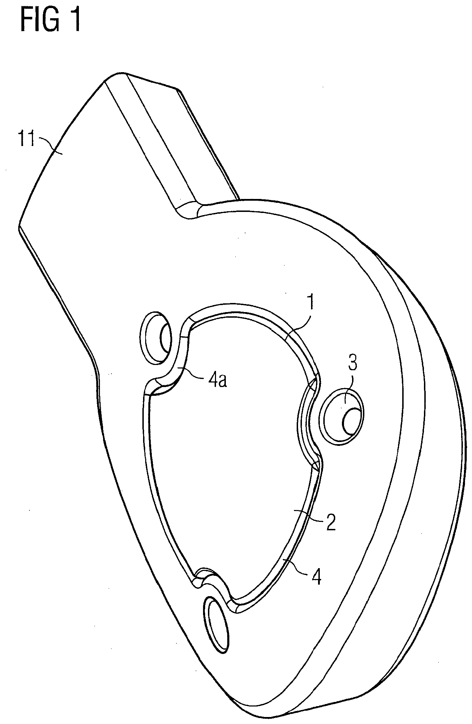

Wie in den Figuren 1 bis 4 gezeigt ist, hat eine Implantatstruktur 11 eines Körperimplantats bevorzugt eine Bogen- bzw. Kreis- bzw. Ellipsen- bzw. Zylinderform, um an der Wandung eines Körperkanals bzw. Hohlraums flächig anzuliegen. Ein Ausschnitt 1 zum Einsetzen eines Markerelements 2, 2a wird vorzugsweise durch Laserschneiden in der Implantatstruktur 11 geformt. Durch das Laserschneiden sowie die zumindest bereichsweise Bogenform der Implantatstruktur 11 erhält der Ausschnitt 1 zum Einsetzen des Markerelements 2, 2a eine konische Form bzw. eine Kegelform.

In diesen Ausschnitt 1 kann ein Markerelement 2, 2a durch Verpressen bzw. Vernieten so eingesetzt werden, daß ein Formschluß des Markenelements 2, 2a gewährleistet ist. Hierdurch wird eine hohe mechanische Festigkeit bzw. Verriegelung des Markerelements 2 in dem Körperimplantat 1 erzielt. Ein Verkleben, Verschweißen etc. wird hierdurch überflüssig, wodurch die Herstellungskosten des Körperimplantats 1 niedrig gehalten werden können.

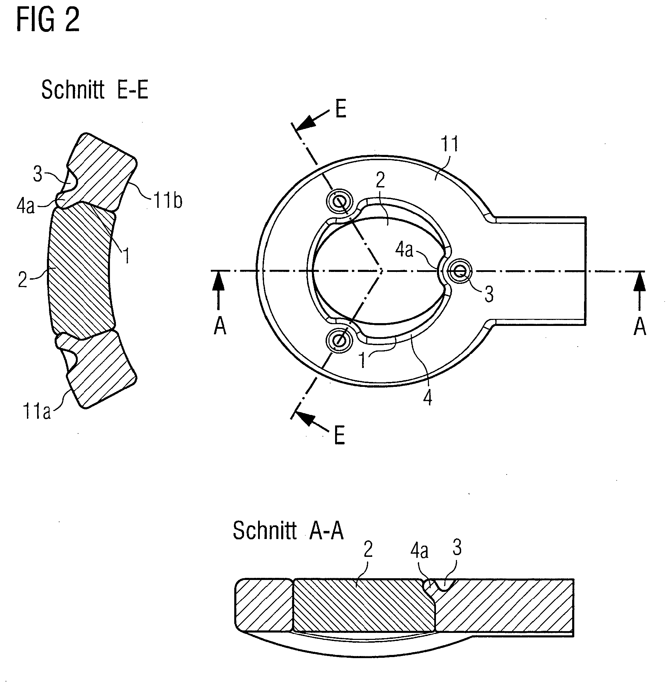

Wie insbesondere in den Figuren 1 und 2 gezeigt ist, wird zum Fixieren des Markerelements 2, 2a der Rand des Ausschnitts 1 mit dem größeren Durchmesser an mehreren Stellen, vorzugsweise an drei Stellen im gleichen Winkelabstand im wesentlichen punktförmig verformt, so dass Material der Implantatstruktur 11 in Richtung zu dem Ausschnitt 1 fließt. Auf diese Weise bildet der verformte Rand 4 zusammen mit der Kegelform des Ausschnitts 1 einen Formschluss mit dem Markerelement 2, 2a, um dieses zu fixieren.

In anderen Worten wird der Rand 4 durch eine Verpressung bereichsweise 3 so verformt, dass der verformte Rand 4a in den Bereich des Ausschnitts 1 fließt, um das eingesetzte Markerelement 2, 2a formschlüssig zu fixieren. Der verformte Rand 4a hat dabei im wesentlichen dieselbe Höhe wie der nicht verformte Rand 4 und es wechseln sich verformte Ränder 4a und nicht verformte Ränder 4 entlang des Umfangs ab.

Die Verpressung 3 zum Bilden des verformten Rands 4a ist vorzugsweise im wesentlichen punktförmig, d.h. wie eine mittels eines Körners erzeugte Körnung ausgebildet, die mittels Eindrücken oder Einprägen eines im wesentlichen spitzen Gegenstands (Körner) in die Implantatstruktur 11 erzeugt wird. Dabei bildet sich eine im wesentlichen kreisrunde Vertiefung an der Stelle der Verpressung 3 durch das Eindringen des spitzen Gegenstands in das Material der Implantatstruktur 11.

Die Erfindung ist jedoch nicht auf das Bilden einer kreisrunden Vertiefung beschränkt, sondern die Vertiefung kann auch eine beliebige andere Querschnittsform haben, um die Verpressung 3 zu bilden. Es ist lediglich erforderlich, durch die Verpressung 3 ein Fließen des Materials der Implantatstruktur 11 derart zu erzeugen, dass der Rand 4 des Ausschnitts 1 verformt wird, um durch den verformten Rand 4a einen Formschluss mit dem Markerelement 2, 2a zu bilden.

Die Verpressung 3 zum Bilden des verformten Rands 4a wird vorzugsweise nur an einer Außenseite 11 a der Implantatstruktur 11 erzeugt, weil das Markerelement 2, 2a durch die Kegelform des Ausschnitts 1 nicht aus der Innenseite 11 b der Implantatstruktur 11 austreten kann. Die Erfindung ist jedoch nicht hierauf beschränkt. Die Verpressung 3 kann auch auf der Innenseite 11b oder auf beiden Seiten, der Innenseite 11 b und der Aussenseite 11a, erzeugt werden.

Darüber hinaus muss der Ausschnitt 1 keine durchgängige Öffnung bilden, sondern kann als Sackloch bzw. Aushöhlung gebildet sein, so dass das Markerelement 2, 2a auf einer Seite, vorzugsweise der Innenseite des Körperimplantats, durch den Boden der Aushöhlung fixiert wird und auf der anderen Seite durch die Verpressung 3 mit dem verformten Rand 4a.

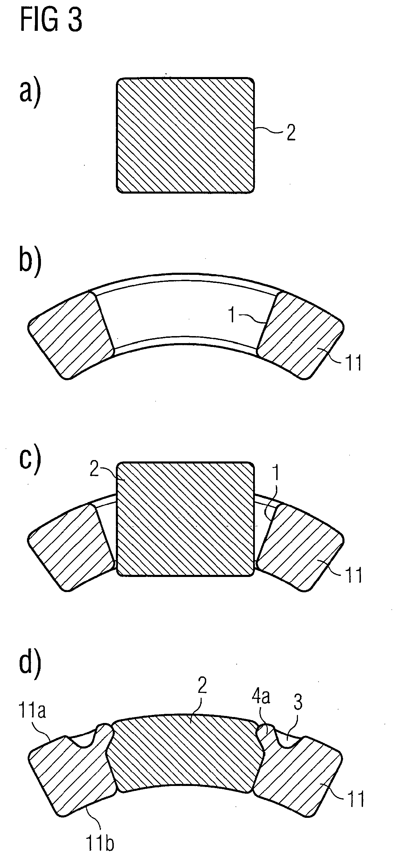

Das Markerelement 2 kann vor dem Einsetzen und Verpressen eine zylindrische Form haben, wie in Fig. 3a gezeigt ist. Durch ein geeignetes Niet- oder Presswerkzeug wird das Markerelement 2 so verpresst, dass es die Gestalt des Ausschnitts 1 annimmt, wie in Fig. 3d gezeigt ist. Danach wird das Markerelement 2 durch Bilden der Verpressung 3 und Erzeugen des verformten Rands 4a in dem Ausschnitt 1 gesichert bzw. fixiert.

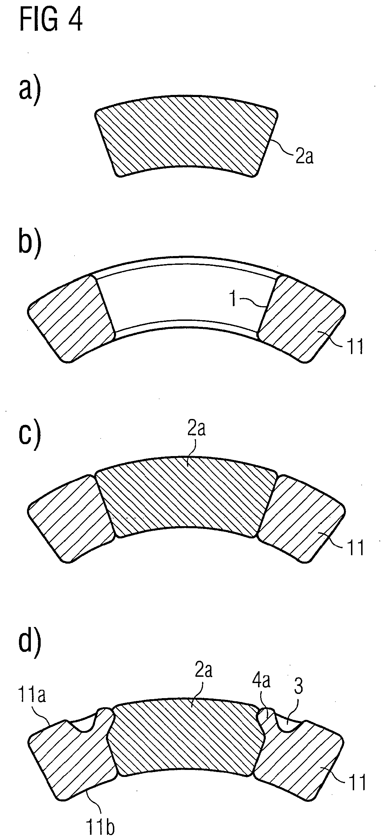

Das Markerelement 2a kann jedoch bereits vor dem Einsetzen in den Ausschnitt 1 dessen Gestalt aufweisen, wie in Fig. 4a gezeigt ist. Ein derart gestaltetes Markerelement 2a wird vorzugsweise dadurch hergestellt, dass es aus einem Rohr ausgeschnitten wird.

Fig. 2, 3d und 4d zeigen schließlich daß verpreßte Markerelement 2, 2a in dem Körperimplantat. Vorzugsweise wird das Markerelement 2 dabei so verpreßt, daß es im wesentlichen mit der Implantatstruktur 11 eine ebene Fläche bildet. Hierdurch werden Kanten an dem Übergang zwischen dem Markerelement 2, 2a und dem Körperimplantat vermieden, die ansonsten zu Verletzungen eines Körperkanals, in den das Körperimplantat eingesetzt wird, führen könnten.

Der Marker 2, 2a weist vorzugsweise ein röntgensichtbares Material, wie beispielsweise Gold, Platin, Tantal, Niob oder eine Platinlegierung, wie beispielsweise Platin-Iridium, auf.

Vorzugsweise ist das Markerelement 2, 2a in seinen Abmessungen so auf den Ausschnitt 1 abgestimmt, daß das Markerelement 2, 2a nach dem Verpressen im wesentlichen innen/außen eben mit der Implantatstruktur 11 ist. Falls jedoch eine erhöhte Anforderung an die Ebenheit gestellt wird, kann das Markerelement 2, 2a auch etwas größer bemessen sein und nach dem Verpressen eine Oberflächenbehandlung, beispielsweise durch einen Laser, Elektropolieren oder Schleifen, erhalten.

Durch die Verpressung 3 zum Erzeugen des verformten Rands 4a wird ein Formschluß zwischen dem Markerelement 2, 2a und der Implantatstruktur 11 gebildet, so dass eine gute Befestigung des Markerelements 2, 2a in der Implantatstruktur 11 gewährleistet ist. Ein Verkleben bzw. Verschweißen kann entfallen, um Fertigungskosten einzusparen. Jedoch kann auch zusätzlich zu dem Formschluß ein Verkleben bzw. Verschweißen des Markerelements 2, 2a erfolgen.

Das Körperimplantat weist bevorzugt eines der folgenden Materialien auf: rostfreier Edelstahl, Kobalt-Chrom-Tantal-Legierung, Polymere, selbstabbaubare Werkstoffe, wie beispielsweise Milchsäure-Werkstoffe bzw. Derivaten, sowie Nitinol (Nickel-Titan-Legierungen) und/oder andere selbstexpandierbare Werkstoffe, wie beispielsweise sogenannte Formgedächtnis-Werkstoffe.

### Bezugszeichenliste

- 1: Ausschnitt
- 2, 2a: Markerelement
- 3: Verpressung
- 4: Rand
- 4a: verformter Rand
- 11: Implantatstruktur
- 11a: Aussenseite
- 11b: Innenseite
- 14: Innenkonus
- 16: Ringspalt
- 18: Ringspalt

## Patentansprüche

1. Körperimplantat, insbesondere Stent zum Einführen oder Implantieren in einen lebenden Körper, mit einem Markerelement (2; 2a) aus einem röntgensichtbaren Material, das in einen Ausschnitt (1) einer Implantatstruktur (11) eingesetzt ist, **dadurch gekennzeichnet, dass** zumindest ein Rand (4) des Ausschnitts (1) bereichsweise derart verformt bzw. verpresst ist, dass das Markerelement (2; 2a) formschlüssig von dem Ausschnitt (1) aufgenommen ist, indem sich verformte Ränder (4a) und nicht verformte Ränder (4) entlang des Umfangs des Ausschnitts (1) abwechseln.

2. Körperimplantat nach Anspruch 1, wobei der Ausschnitt (1) im wesentlichen konisch ist und der Rand (4) mit dem größeren Durchmesser verpresst ist, um das Markerelement (2; 2a) zu fixieren.

3. Körperimplantat nach einem der vorherigen Ansprüche, wobei der Rand (4) an mehreren Stellen im wesentlichen punktförmig verformt ist.

4. Körperimplantat nach einem der vorherigen Ansprüche, wobei das Markerelement (2; 2a) vor einem Einpressen im wesentlichen zylindrisch ist und nach dem Einpressen im Querschnitt eine Kreisbogenform in Übereinstimmung mit dem Ausschnitt (1) aufweist oder vor dem Einpressen bereits die Kreisbogenform aufweist.

5. Körperimplantat nach einem der vorherigen Ansprüche, wobei das Markerelement (2; 2a) nach dem Einpressen bzw. Einnieten innen/außen im wesentlichen eben mit der Implantatstruktur (11) ist.

6. Körperimplantat nach einem der vorherigen Ansprüche, wobei das Markerelement (2; 2a) ein Material mit hoher Absorption für Röntgenstrahlen wie beispielsweise Gold, Platin, Tantal, Platinlegierung, Platin-Iridium, Niob aufweist.

7. Verfahren zum Herstellen eines Körperimplantats, insbesondere eines Stents, mit den Schritten:
Formen eines Ausschnitts (1) in einer Implantatstruktur (11);
Einsetzen eines Markerelements (2; 2a) in den Ausschnitt (1); und
**gekennzeichnet durch** den Schritt:
Verformen bzw. Verpressen (3) eines Rands (4) des Ausschnitts (1) bereichsweise derart, dass das Markerelement (2; 2a) formschlüssig von dem Ausschnitt (1) aufgenommen wird, indem sich verformte Ränder (4a) und nicht verformte Ränder (4) entlang des Umfangs des Ausschnitts (1) abwechseln.

8. Verfahren nach Anspruch 7, des weiteren mit dem Schritten:
Formen des Ausschnitts (1) im wesentlichen in einer konischen Gestalt; und
Verpressen des Rands (4) mit dem größeren Durchmesser, um das Markerelement (2; 2a) zu fixieren.

9. Verfahren nach Anspruch 7 oder 8, des weiteren mit dem Schritt des punktförmigen Verformens (3) des Rands (4) an mehreren Stellen, vorzugsweise an drei Stellen in im wesentlichen gleichen Winkelabständen.

10. Verfahren nach einem der Ansprüche 7 bis 9, des weiteren mit dem Schritt des Ausschneidens des Markerelements (2a) aus einem Rohr.

## Claims

1. A body implant, particularly a stent, for introducing or implanting into a living body, comprising a marker element (2; 2a) of an x-ray visible material inserted into a cutout (1) of an implant structure (11), **characterized in that** at least a rim (4) of the cutout (1) is deformed or compressed in part(s) such that the marker element (2; 2a) is positively received by the cutout (1) by deformed rims (4a) and non-deformed rims (4) alternating along the circumference of the cutout (1).

2. The body implant according to claim 1, wherein the cutout (1) is substantially conical and the rim (4) with the larger diameter is compressed to fix the marker element (2; 2a).

3. The body implant according to one of the preceding claims, wherein the rim (4) is deformed substantially in a punctiform manner at several points.

4. The body implant according to one of the preceding claims, wherein the marker element (2; 2a) is substantially cylindrical before being pressed-in and has a circular arc-shaped cross-section in correspondence with the cutout (1) after being pressed-in, or already has the circular arc shape before being pressed-in.

5. The body implant according to one of the preceding claims, where the marker element (2; 2a) is substantially level with the implant structure (11) on the inside/outside after being pressed-in or riveted.

6. The body implant according to one of the preceding claims, wherein the marker element (2; 2a) has a material with high absorption for x-rays, such as gold, platinum, tantalum, platinum alloy, platinum iridium, niobium.

7. A method of producing a body implant, particularly a stent, comprising the steps of:
forming a cutout (1) in an implant structure (11);
inserting a marker element (2; 2a) into the cutout (1); and
**characterized by** the step of:
deforming or compressing (3) a rim (4) of the cutout (1) in part(s) such that the marker element (2; 2a) is positively received by the cutout (1) by deformed rims (4a) and non-deformed rims (4) alternating along the circumference of the cutout (1).

8. The method according to claim 7, further comprising the steps of:
forming the cutout (1) substantially in a conical shape, and
compressing the rim (4) with the larger diameter to fix the marker element (2; 2a).

9. The method according to claim 7 or 8, further comprising the step of puncti-formly deforming (3) the rim (4) at several points, preferably at three pints at substantially equal angular distances.

10. The method according to one of claims 7 to 9, further comprising the step of cutting the marker element (2a) out of a pipe.

## Revendications

1. Implant corporel, notamment stent pour l'introduction ou l'implantation dans un corps vivant, avec un élément marqueur (2 ; 2a) en un matériau visible aux rayons X qui est inséré dans une découpe (1) d'une structure d'implant (11), **caractérisé en ce qu'**au moins un bord (4) de la découpe (1) est déformé ou comprimé par région de telle sorte que l'élément marqueur (2 ; 2a) est reçu par conjugaison de formes par la découpe (1) **en ce que** des bords déformés (4a) et des bords non déformés (4) alternent le long de la périphérie de la découpe (1).

2. Implant corporel selon la revendication 1, dans lequel la découpe (1) est essentiellement conique et le bord (4) avec le diamètre supérieur est comprimé pour fixer l'élément marqueur (2 ; 2a).

3. Implant corporel selon une des revendications précédentes, dans lequel le bord (4) est déformé essentiellement en forme de point à plusieurs endroits.

4. Implant corporel selon une des revendications précédentes, dans lequel l'élément marqueur (2 ; 2a) est essentiellement cylindrique avant un enfoncement et présente une forme d'arc de cercle en coupe transversale en concordance avec la découpe (1) après l'enfoncement ou présente déjà la forme d'arc de cercle avant l'enfoncement.

5. Implant corporel selon une des revendications précédentes, dans lequel l'élément marqueur (2 ; 2a) est essentiellement plan avec la structure d'implant (11) à l'intérieur/extérieur après l'enfoncement ou le rivetage.

6. Implant corporel selon une des revendications précédentes, dans lequel l'élément marqueur (2 ; 2a) présente un matériau à haute absorption aux rayons X comme par exemple de l'or, du platine, du tantale, un alliage de platine, du platine-iridium, du niobium.

7. Procédé de fabrication d'un implant corporel, notamment d'un stent, avec les étapes :
façonnage d'une découpe (1) dans une structure d'implant (11) ;
insertion d'un élément marqueur (2 ; 2a) dans la découpe (1) ; et
**caractérisé par** l'étape :
déformation ou compression (3) d'un bord (4) de la découpe (1) par région de telle sorte que l'élément marqueur (2 ; 2a) est reçu par conjugaison de formes par la découpe (1) en ce que des bords déformés (4a) et des bords non déformés (4) alternent le long de la périphérie de la découpe (1).

8. Procédé selon la revendication 7, en outre avec les étapes :
façonnage de la découpe (1) essentiellement dans une configuration conique ; et
compression du bord (4) avec le diamètre supérieur pour fixer l'élément marqueur (2 ; 2a).

9. Procédé selon la revendication 7 ou 8, en outre avec l'étape de la déformation en forme de point (3) du bord (4) à plusieurs endroits, de préférence à trois endroits à des écarts angulaires essentiellement égaux.

10. Procédé selon une des revendications 7 à 9, en outre avec l'étape de la découpe de l'élément marqueur (2a) d'un tuyau.
